# EUROPÄISCHE PATENTSCHRIFT

(11) **EP 1 172 355 B1**
(45) Veröffentlichungstag und Bekanntmachung des Hinweises auf die Patenterteilung: **27.08.2003**
(21) Anmeldenummer: 01115054.7
(22) Anmeldetag: 21.06.2001
(51) Int. Cl.: C07C 251/86, A01N 33/26

(54) **Salicylsäure-Derivate, Verfahren zu ihrer Herstellung, sie enthaltende Mittel sowie ihre Verwendung zur Bekämpfung von Schadpilzen**
Salicylic acid derivatives, process for their preparation, agents containing the same as well as their use in combatting harmful fungi
Dérivés de l'acide salicylique, procédé pour leur préparation, agents les contenant ainsi que leur utilisation pour lutter contre les champignons nuisibles

(30) Priorität: 13.07.2000 DE 10034180
(43) Veröffentlichungstag der Anmeldung: 16.01.2002
(73) Patentinhaber: BASF AKTIENGESELLSCHAFT, 67056 Ludwigshafen (DE)
(72) Erfinder: Gypser, Andreas, Dr., 68159 Mannheim (DE); Grote, Thomas, Dr., 67157 Wachenheim (DE); Rheinheimer, Joachim, Dr., 67063 Ludwigshafen (DE); Rose, Ingo, Dr., 68159 Mannheim (DE); Cullmann, Oliver, Dr., 64646 Heppenheim (DE); Gewehr, Markus, Dr., 56288 Kastellaun (DE); Grammenos, Wassilios, Dr., 67063 Ludwigshafen (DE); Tormo i Blasco, Jordi, Dr., 67117 Limburgerhof (DE); Müller, Bernd, Dr., 67227 Frankenthal (DE); Sauter, Hubert, Dr., 68167 Mannheim (DE); Ammermann, Eberhard, Dr., 64646 Heppenheim (DE); Strathmann, Siegfried, Dr., 67117 Limburgerhof (DE); Lorenz, Gisela, Dr., 67434 Hambach (DE); Stierl, Reinhard, Dr., 67112 Mutterstadt (DE)

(56) Entgegenhaltungen:
- WO-A-97/08135
- US-A- 3 829 492
- A.I. EL-SEBAI ET AL.: "Synthesis of some new acid hydrazides structurally related to certain tuberculostatic agents." EGYPT. J. PHARM. SCI.,, Bd. 14, Nr. 1, 1973, Seiten 67-73, XP000926689

## Beschreibung

Die vorliegende Erfindung betrifft Salicylsäure-Derivate der Formel I, 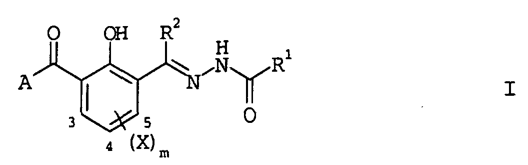 in der der Index und die Substituenten folgende Bedeutung haben:
- X: Halogen, NO₂, Cyano, C₁-C₄-Alkyl oder C₁-C₄-Alkoxy;
- m: 0, 1, 2 oder 3, wobei die Substituenten X verschieden sein können, wenn m größer als 1 ist;
- A: OH, C₁-C₄-Alkoxy, NH₂, NHCH₃ oder N(CH₃)₂;
- R¹: Phenyl, welches unsubstituiert oder substituiert ist durch einen bis drei Reste R^{a}:
R^{a} Cyano, Nitro, Amino, Aminocarbonyl, Aminothiocarbonyl, Halogen, Hydroxy, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Phenyl, Phenoxy, Benzyl, Benzyloxy, 5- oder 6-gliedriges Heterocyclyl, 5- oder 6-gliedriges Hetaryl, 5- oder 6-gliedriges Hetaryloxy, C(=NOR^{α})-OR^{β} oder OC(R^{α})₂-C(R^{β})=NOR^{β},
wobei die cyclischen Reste ihrerseits unsubstituiert oder substituiert sind durch einen bis drei Reste R^{b}:
R^{b} Cyano, Nitro, Halogen, Hydroxy, Amino, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkenyl, Phenyl, Phenoxy, Phenylthio, Benzyl, Benzyloxy, 5- oder 6-gliedriges Heterocyclyl, 5- oder 6-gliedriges Hetaryl, 5- oder 6-gliedriges Hetaryloxy oder C(=NOR^{α})-OR^{β};
R^{α},R^{β} Wasserstoff oder C₁-C₆-Alkyl;
- R²: Wasserstoff, Cyano, C₁-C₆-Alkyl, C₂-C₆-Alkenyl, C₂-C₆-Alkinyl, C₁-C₆-Halogenalkyl, C₁-C₆-Alkoxy oder C₁-C₆-Alkylthio,
wobei die Kohlenwasserstoffreste unsubstituiert oder partiell oder vollständig halogeniert sind oder eine bis drei Gruppen R^{c} tragen können:
R^{c} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino,
Di-C₁-C₆-alkylamino, C₂-C₆-Alkenyl, C₃-C₆-Alkenyloxy, C₃-C₆-Alkinyloxy oder C₁-C₄-Alkylendioxy, welches halogeniert sein kann; mit Ausnahme von 3-[(4-Amino-2-hydroxy-benzoyl)-hydrazonomethyl]-2-hydroxy-Benzoesäure.

Außerdem betrifft die Erfindung Verfahren zur Herstellung dieser Verbindungen, sie enthaltende Mittel sowie ihre Verwendung zur Bekämpfung von Schadpilzen.

In US 3,829,492 werden fungizid wirksame Salicylaldehydhydrazone offenbart.

In Egypt. J. Pharm. Sci., Bd. 14, S. 67 ff. (1973) sind pharmazeutisch wirksame Isocotinylhydrazine beschrieben.

Aus WO-A 97/08135, DE-A 197 10 609 und WO-A 99/27783 sind Acylamino-Salicylsäureamide zur Bekämpfung von Schadpilzen bekannt.

Deren Wirkung kann jedoch in vielen Fällen nicht befriedigen. Der vorliegenden Erfindung lagen Verbindungen mit verbesserter Wirkung als Aufgabe zugrunde.

Demgemäß wurden die eingangs definierten Verbindungen gefunden. Desweiteren wurden Verfahren zu ihrer Herstellung, sie enthaltende Mittel sowie Verfahren zur Bekämpfung von pflanzenpathogenen Schadpilzen unter Verwendung der Verbindungen I gefunden.

Die Verbindungen der Formel I unterscheiden sich von den aus dem Stand der Technik bekannten durch die Hydrazid-Gruppierung.

Die Verbindungen der Formel I weisen eine gegenüber den bekannten Verbindungen erhöhte Wirksamkeit gegen Schadpilze auf.

Verbindungen der Formel I können beispielsweise ausgehend von Salicylsäurealkylestern der Formel II, in der A für C₁-C₄-Alkoxy und Z für eine unter sauren Bedingungen abspaltbare Schutzgruppe steht, wie beispielsweise eine Acetylgruppe, hergestellt werden.

Verbindungen der Formel II können durch durch radikalische Halogenierung unter allgemein üblichen Bedingungen, besonders vorteilhaft durch Bromierung mit N-Bromsuccinimid (NBS) unter Verwendung von Azobisisobutyronitril (AIBN) als Radikalstarter unter Belichtung erhalten werden [vgl. J. Amer. Chem. Soc., Bd. 71, S. 2137ff. (1949); ebd., Bd. 90, S. 1797ff. (1968)]. Verbindungen der Formel II, in der A für Methoxy, Z für Acetyl und Hal für Brom steht, sind bevorzugt.

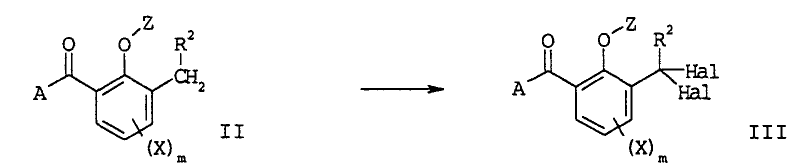

Diese Umsetzung erfolgt üblicherweise bei Temperaturen von 20°C bis 150°C, vorzugsweise 50°C bis100°C, in einem inerten organischen Lösungsmittel [vgl. Can. J. Chem., Bd. 33, S. 1819 (1955); J. Org. Chem., Bd. 49, S. 2158 (1984)].

Geeignete Lösungsmittel sind halogenierte Kohlenwasserstoffe wie Tetrachlorkohlenstoff, Chloroform und Chlorbenzol. Es können auch Gemische der genannten Lösungsmittel verwendet werden.

Die Edukte werden im allgemeinen in äquimolaren Mengen miteinander umgesetzt. Es kann für die Ausbeute vorteilhaft sein, NBS in einem Überschuß bezogen auf II einzusetzen.

Die für die Herstellung der Verbindungen I benötigten Ausgangsstoffe II sind kommerziell erhältlich, in der Literatur bekannt [Bull. Soc. Chim. Fr., Bd. 9, S. 2821 (1966); Biotechnol. Lett., Bd. 15, S. 469 (1993); J. Fluorine Chem., Bd. 74, S. 69 (1995)] oder können gemäß der zitierten Literatur hergestellt werden.

Die Dihalogenverbindungen der Formel III werden unter Abspaltung der Schutzgruppe Z unter sauren Bedingungen zu den Ketoverbindungen IV oxidiert.

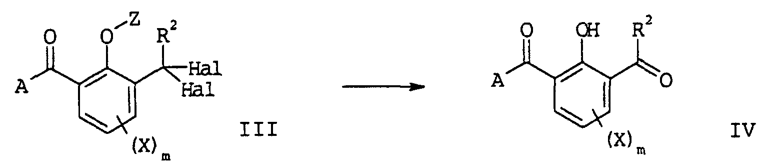

Diese Umsetzung erfolgt üblicherweise bei Temperaturen von 20°C bis 150°C, vorzugsweise 20°C bis 100°C, in einem inerten organischen Lösungsmittel in Gegenwart einer Säure [vgl. Org. Synth., Bd. 20, S. 92 (1940)].

Geeignete Lösungsmittel sind aliphatische Kohlenwasserstoffe, aromatische Kohlenwasserstoffe, halogenierte Kohlenwasserstoffe, Ether, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Methanol, Ethanol und n-Propanol. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Toluolsulfonsäure, Benzolsulfonsäure, Camphersulfonsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Zur Herstellung von Verbindungen I, in der A für NH₂, NHCH₃ oder N(CH₃)₂ steht, werden Salicylsäureester IV mit Ammoniak oder Methylamin zu Salicylsäureamiden der Formel IV.1, in der R³ für Wasserstoff oder Methyl steht, umgesetzt; die Umsetzung mit Dimethylamin führt zu Verbindungen der Formel IV.3:

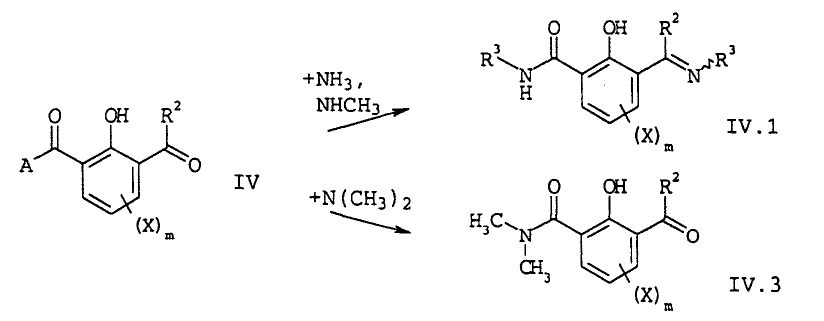

Diese Umsetzung erfolgt üblicherweise bei Temperaturen von 20°C bis 150°C, vorzugsweise 20°C bis 100°C, in einem inerten organischen Lösungsmittel [vgl. Arch. Pharm., S. 941 (1982)].

Geeignete Lösungsmittel sind aliphatische Kohlenwasserstoffe, aromatische Kohlenwasserstoffe, halogenierte Kohlenwasserstoffe, Ether wie Diethylether, Diisopropylether, tert.-Butylmethylether, Dioxan, Anisol und Tetrahydrofuran, Nitrile wie Acetonitril, Alkohole, sowie Dimethylsulfoxid, Dimethylformamid und Dimethylacetamid, besonders bevorzugt Dimethylformamid und Dimethylacetamid. Es können auch Gemische der genannten Lösungsmittel verwendet werden.

Die Edukte werden im allgemeinen in äquimolaren Mengen miteinander umgesetzt. Es kann für die Ausbeute vorteilhaft sein, das Amin in einem Überschuß bezogen auf IV einzusetzen.

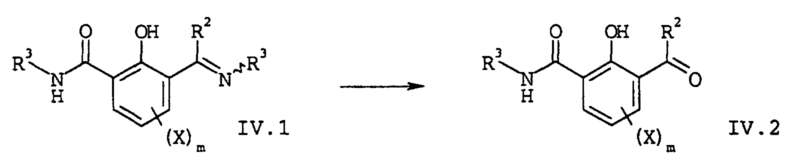

Die Abspaltung der Iminogruppe aus IV.1 erfolgt üblicherweise bei Temperaturen von 20°C bis 150°C, vorzugsweise 20°C bis 100°C, in einem inerten organischen Lösungsmittel in Gegenwart einer Säure [vgl. J. Chem. Soc., S. 3807 (1957); J. Org. Chem., Bd. 6, S. 489 (1941)].

Geeignete Lösungsmittel sind aliphatische Kohlenwasserstoffe, aromatische Kohlenwasserstoffe, halogenierte Kohlenwasserstoffe, Ether, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Methanol, Ethanol und n-Propanol. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure. Essigsäure, Propionsäure, Oxalsäure, Toluolsulfonsäure, Benzolsulfonsäure, Camphersulfonsäure, Zitronensäure und Trifluoressigsäure Verwendung.

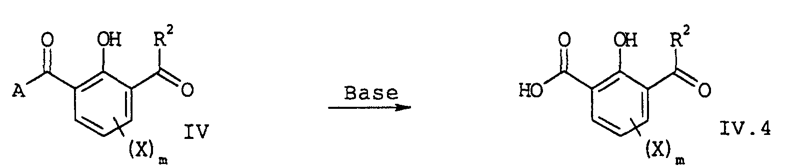

Zur Herstellung von Verbindungen der Formel I, in der A für Hydroxy steht, werden die Alkylester der Formel IV nach üblichen Methoden zu den Salicylsäuren der Formel IV.4 verseift [vgl. Organikum, 16. Aufl., S. 415 und 622, VEB Deutscher Verlag der Wissenschaften, Berlin 1985]. Diese Reaktion erfolgt üblicherweise bei Temperaturen von 10°C bis 80°C, vorzugsweise 20°C bis 60°C, in einem inerten organischen Lösungsmittel in Gegenwart einer Base, wie Alkali- oder Erdalkalihydroxiden, insbesondere Natrium- oder Kaliumhydroxid.

Je nach Ausgestaltung der Restes A in Formel I werden Verbindungen der Formeln IV, IV.2, IV.3 oder IV.4 mit Hydraziden der Formel V zu Verbindungen der Formel I umgesetzt.

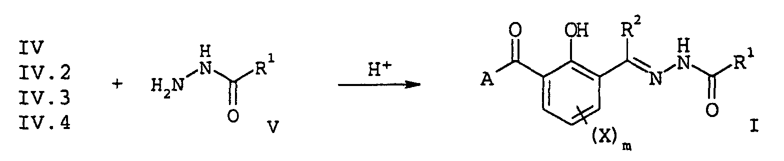

Diese Umsetzung erfolgt üblicherweise bei Temperaturen von 20°C bis 150°C, vorzugsweise 20°C bis 80°C, in einem inerten organischen Lösungsmittel in Gegenwart einer Säure [vgl. Org. Prep. Proced. Int., Bd. 30, S. 177 (1998); CH-A 661 276].

Geeignete Lösungsmittel sind aliphatische Kohlenwasserstoffe, aromatische Kohlenwasserstoffe, halogenierte Kohlenwasserstoffe, Ether, Nitrile, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Methanol, Ethanol, n-Propanol, Isopropanol oder Dimethylformamid. Es können auch Gemische der genannten Lösungsmittel verwendet werden.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Toluolsulfonsäure, Benzolsulfonsäure, Camphersulfonsäure, Zitronensäure und Trifluoressigsäure Verwendung.

Die Säuren werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, V in einem Überschuß bezogen auf IV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Die Verbindungen I können bei der Herstellung aufgrund ihrer C=Cund C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4 oder 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Alkoxy:** geradkettige oder verzweigte Alkylgruppen mit 1 bis 6 Kohlenstoffatomen (wie vorstehend genannt), welche über ein Sauerstoffatom (-O-) an das Gerüst gebunden sind;
**Alkylen:** divalente unverzweigte Ketten aus 1 bis 4 CH₂-Gruppen, z.B. CH₂, CH₂CH₂, CH₂CH₂CH₂ und CH₂CH₂CH₂CH₂ ;
**Alkylendioxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O.

Im Hinblick auf ihre bestimmungsgemäße Verwendung der Salicylsäure-Derivate der Formel I sind die folgenden Bedeutungen der Substituenten, und zwar jeweils für sich allein oder in Kombination, besonders bevorzugt:
Verbindungen der Formel I, in der A für Methyl, Methoxy, Hydroxy, Amino und Methylamino steht, sind besonders bevorzugt.

Insbesondere werden Verbindungen IA bevorzugt:

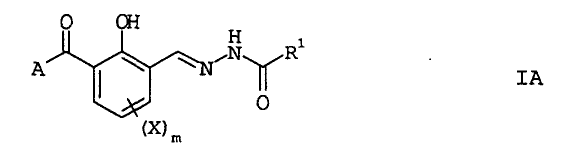

Daneben werden Verbindungen I besonders bevorzugt, in denen der Index m Null bedeutet; Diese Verbindungen werden durch die Formel IB beschrieben:

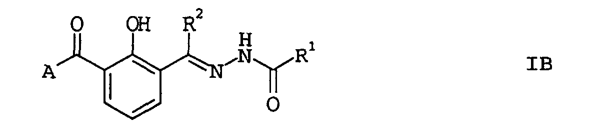

Weiterhin werden Verbindungen I besonders bevorzugt, in denen R² Methyl bedeutet.

Außerdem werden Verbindungen I besonders bevorzugt, in denen (X)ₘ para-ständig zu der Phenol-OH-Gruppe steht und Halogen, NO₂, CN und C₁-C₄-Alkoxy, insbesondere 4-Chlor, 4-Brom und 4-NO₂, bedeutet.

Ebenfalls bevorzugt sind Verbindungen I, in denen R^{a} Chlor, Brom, Nitro, Cyano, Methyl, Ethyl, n- und iso-Propyl, n-, sek.- und tert.-Butyl und Phenyl, wobei der aromatische Ring unsubstituiert oder durch einen oder zwei Reste aus der Gruppe Chlor, Brom, Nitro, Cyano und Methyl substituiert ist, bedeutet.

Gleichermaßen besonders bevorzugt sind Verbindungen I, in denen R² für Wasserstoff, Methyl, Methoxy und Cyano, insbesondere für Wasserstoff und Methyl steht.

Die besonders bevorzugten Ausführungsformen der Zwischenprodukte in Bezug auf die Variablen entsprechen denen der Reste A, (X)ₘ, R¹, und R² der Formel I.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon. Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz. 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino )-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl] -piperidin, 1- [2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino- [a- (2-phenoxyphenyl)]-acetamid, Methyl-E-methoxyimino-[a-(2, 5-dimethylphenoxy)-o-tolyl]-acetamid, Methyl-E-2-{2-[2-trifluormethylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl] -glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl )-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino)-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1 - Herstellung von 3 -(1,1-Dibrom)methyl-acetoxysalicylsäuremethylester

53 g (0,25 mol) 3-Methyl3-Methyl-acetoxysalicylsäuremethylester, 95 g (0,53 mol) N-Bromsuccinimid und eine Spatelspitze Azobisisobutyronitril (AIBN) als Radikalstarter wurden in 600 ml Tetrachlorkohlenstoff mit einer UV-Lampe (500 W) bestrahlt. Die Reaktionmischung erwärmte sich für etwa 6 min auf Rückfluß. Nach weiteren 15 min Bestrahlung liess man auf Raumtemperatur abkühlen. Das ausgefallene Succinimid wurde abfiltriert und mit CCl₄ gewaschen. Das Filtrat wurde vom Lösungsmittel befreit. Nach Chromatographie an Kieselgel (Cyclohexan:Methyl-tert.-butylether [MTBE] 5:1) erhielt man 72 g der Titelverbindung, was 78% der Theorie entspricht.

### Beispiel 2 - Herstellung von 3-Formyl-2-hydroxy-benzoesäuremethylester

Zu einer Lösung von 64 g (0,17 mol) des Dibromids aus Beispiel 1 in 500 ml wasserfr. Methanol wurden bei etwa 20 bis 25°C 63 ml konz. Schwefelsäure zugetropft. Die Temperatur der Reaktionlösung wurde durch Kühlung bei 30°C gehalten. Nach 30 min Rühren bei 20 bis 25°C für 3 Std. auf 50°C aufgeheizt. Das Reaktionsgemisch wurde in etwa 3,5 l Wasser eingerührt. Der Niederschlag wurde abfiltriert und mit Wasser , dann mit Pentan/MTBE-Gemisch (5:1) gewaschen. Nach Trocknen wurden 25 g der Titelverbindung erhalten, was 81% der Theorie etspricht. (Schmp. 82°C)

### Beispiel 3 - Herstellung von 3-Methyliminomethyl-2-hydroxy-benzoesäuremethylamid

Eine Lösung von 12 g (65 mmol) des Aldehyds aus Beispiel 2 in 100 ml Tetrahydrofuran (THF) wurde mit 50 g 40 %iger wässr. Methylaminlösung versetzt. Nach 2 Std. Rühren bei 50°C wurde das Lösungsmittel abdestilliert. Man erhielt 14 g der Titelverbindung, was 99% der Theorie entspricht.

### Beispiel 4 - Herstellung von 3-Formyl-2-hydroxy-benzoesäuremethylamid

14 g (65 mmol) des Methylamids aus Beispiel 3 in einem Gemisch aus 120 ml THF und 100 ml Wasser wurden bei 20 bis 25°C mit 60 ml konz. Salzsäure versetzt. Nach etwa 14 Std. Rühren bei 20 bis 25°C wurde das Reaktionsgemisch in MTBE aufgenommen und mit Wasser gewaschen. Nach Trocknen wurde das Lösungsmittel abdestilliert. Aus dem Rückstand erhielt man 8 g der Titelverbindung, was 71% der Theorie entspricht. (Smp.: 129-131°C)

### Beispiel 5 - Herstellung von 2-Hydroxy-3-(p-tolyl-hydrazonomethyl)-benzoesäuremethylester [I-2]

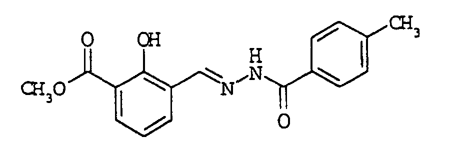

Eine Lösung von 1,1 g (6 mmol) des Aldehyds aus Beispiel 2 in 30 ml wasserfr. Methanol wurde mit 0,9 g (6 mmol) p-Toluolbenzoesäurehydrazid unter Säurekatalyse (para-Toluolsulfonsäure) umgesetzt. Nach etwa 14 Std. Rühren bei 20 bis 25°C wurde das Reaktionsgemisch vom Lösungsmittel befreit. Aus dem Rückstand erhielt man 1,9 g der Titelverbindung, was 99% der Theorie entspricht. (Smp.: 134-137°C)

### Beispiel 6 - Herstellung von 2-Hydroxy-3-(p-tolyl-hydrazonomethyl)-benzoesäuremethylamid [I-13]

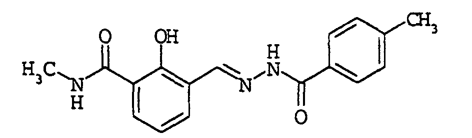

Ein Lösung von 200 mg (1 mmol) des Aldehyds aus Beispiel 4 in 30 ml wasserfr. Methanol wurde mit 140 mg (1 mmol) p-Toluolbenzoesäurehydrazid unter Säurekatalyse (para-Toluolsulfonsäure) umgesetzt. Nach etwa 14 Std. Rühren bei 20 bis 25°C wurde das Reaktionsgemisch vom Lösungsmittel befreit. Aus dem Rückstand erhielt man 340 mg der Titelverbindung, was 99% der Theorie entspricht. (Smp.: 108-125°C)

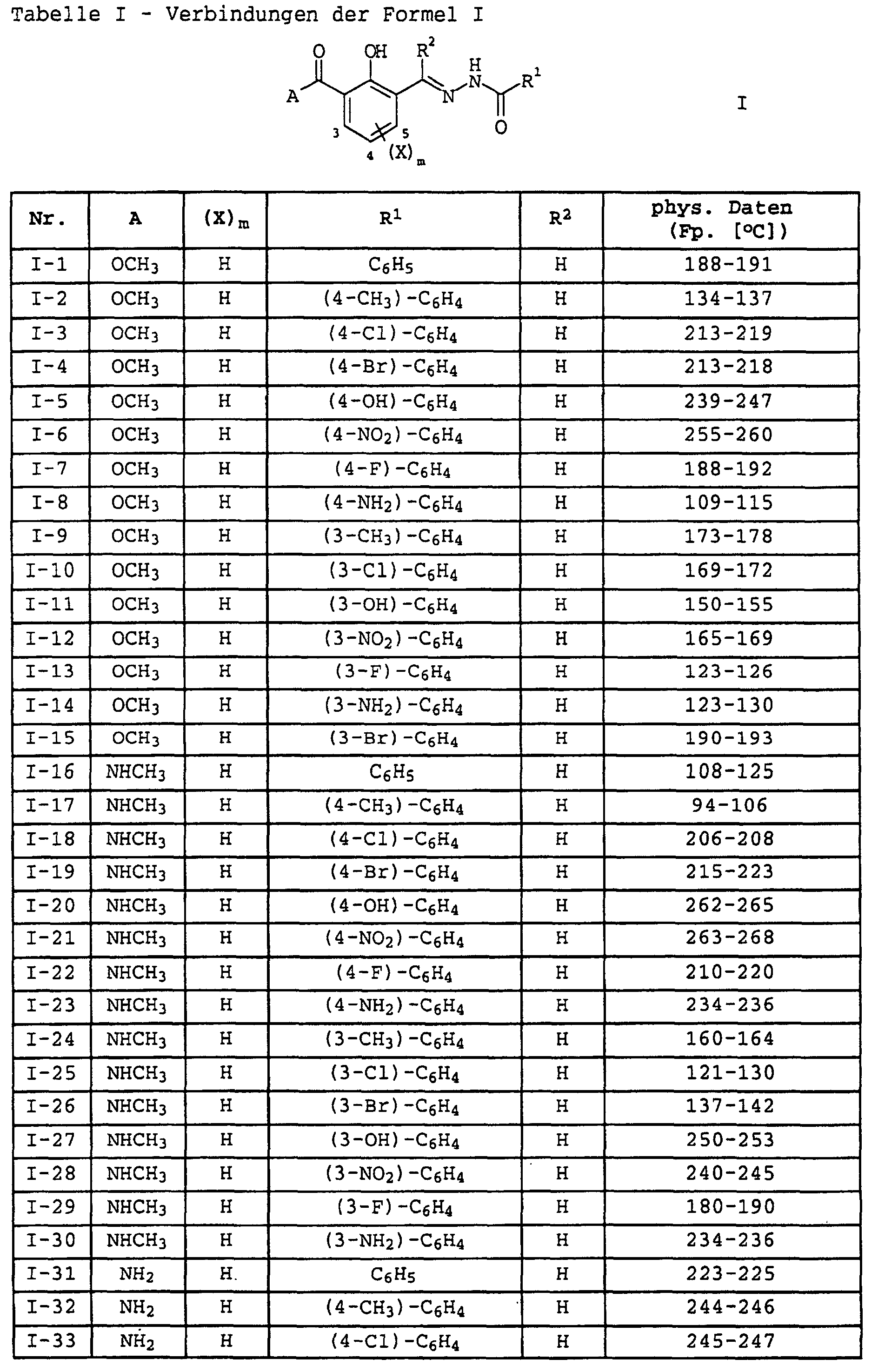

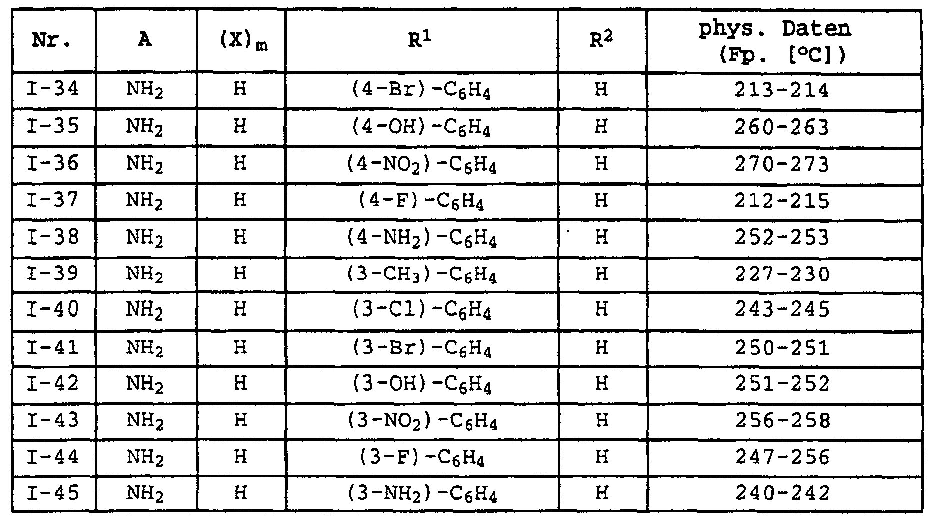

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen Alternaria solani an Tomaten

Blätter von Topfpflanzen der Sorte "Große Fleischtomate St. Pierre" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer wäßrigen Zoosporenaufschwemmung von Alternaria solani in 2 % Biomalzlösung mit einer Dichte von 0.17 x 10⁶ Sporen/ml infiziert. Anschließend wurden die Pflanzen in einer wasserdampfgesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 250 ppm der Wirkstoffe Nr. I-1, I-2, I-3, I-14, I-26 und I-31 der Tabelle I behandelten Pflanzen nicht über 15 % Befall, während die unbehandelten Pflanzen zu 90 % befallen waren.

### Anwendungsbeispiel 2 - Wirksamkeit gegen Botrytis cinerea an Paprikablättern

Paprikasämlinge der Sorte "Neusiedler Ideal Elite" wurden, nachdem sich 4 - 5 Blätter gut entwickelt hatten, mit einer wäßrigen Wirkstoffaufbereitung, die aus einer Stammlösung aus 10 % Wirkstoff, 63 % Cyclohexanon und 27 % Emulgiermittel angesetzt wurde, bis zur Tropfnässe besprüht. Am nächsten Tag wurden die behandelten Pflanzen mit einer Sporensuspension von Botrytis cinerea, die 1.7 x 10⁶ Sporen/ml in einer 2 %igen wäßrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefall auf den Blättern visuell in % ermittelt werden.

In diesem Test zeigten die mit 250 ppm der Wirkstoffe Nr. I-1 bis I-4, I-7, I-9, I-14 und I-25 der Tabelle I behandelten Pflanzen nicht über 20 % Befall, während die unbehandelten Pflanzen zu 100 % befallen waren.

### Anwendungsbeispiel 3 - Wirksamkeit gegen Phytophthora infestans an Tomaten

Blätter von Topfpflanzen der Sorte "Große Fleischtomate St. Pierre" wurden mit einer wäßrigen Suspension, die aus einer Stammlösung aus 10 % Wirkstoff, 63 % Cyclohexanon und 27 % Emulgiermittel angesetzt wurde, bis zur Tropfnässe besprüht. Am folgenden Tag wurden die Blätter mit einer kalten wäßrigen Zoosporenauf-schwemmung von Phytophthora infestans mit einer Dichte von 0.25 x 10⁶ Sporen/ml infiziert. Anschließend wurden die Pflanzen in einer wasserdampfgesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 250 ppm der Wirkstoffe Nr. I-1, I-2, I-7, I-9, I-10, I-11 und I-13 der Tabelle I behandelten Pflanzen nicht über 20 % Befall, während die unbehandelten Pflanzen zu 80 % befallen waren.

## Patentansprüche

1. Salicylsäure-Derivate der Formel I, 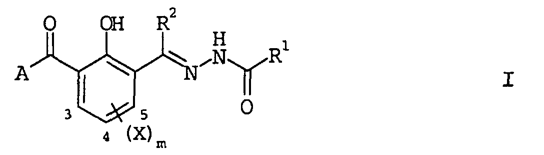 in der der Index und die Substituenten folgende Bedeutung haben:
X Halogen, NO₂, Cyano, C₁-C₄-Alkyl oder C₁-C₄-Alkoxy;
m 0, 1, 2 oder 3, wobei die Substituenten X verschieden sein können, wenn m größer als 1 ist;
A OH, C₁-C₄-Alkoxy, NH₂, NHCH₃ oder N(CH₃)₂;
R¹ Phenyl, unsubstituiert oder substituiert ist durch einen bis drei Reste R^{a}:
R^{a} Cyano, Nitro, Amino, Aminocarbonyl, Aminothiocarbonyl, Halogen, Hydroxy, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Phenyl, Phenoxy, Benzyl, Benzyloxy, 5- oder 6-gliedriges Heterocyclyl, 5- oder 6-gliedriges Hetaryl, 5- oder 6-gliedriges Hetaryloxy, C(=NOR^{α})-OR^{β} oder OC (R^{α})₂-C (R^{β})=NOR^{β},
wobei die cyclischen Reste ihrerseits unsubstituiert oder substituiert sind durch einen bis drei Reste R^{b}:
R^{b} Cyano, Nitro, Halogen, Hydroxy, Amino, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkenyl, Phenyl, Phenoxy, Phenylthio, Benzyl, Benzyloxy, 5- oder 6-gliedriges Heterocyclyl, 5-oder 6-gliedriges Hetaryl, 5- oder 6-gliedriges Hetaryloxy oder C(=NOR^{α})-OR^{β}; R^{α},R^{β} Wasserstoff oder C₁-C₆-Alkyl;
R² Wasserstoff, Cyano, C₁-C₆-Alkyl, C₂-C₆-Alkenyl, C₂-C₆-Alkinyl, C₁-C₆-Halogenalkyl, C₁-C₆-Alkoxy oder C₁-C₆-Alkylthio,
wobei die Kohlenwasserstoffreste unsubstituiert oder partiell oder vollständig halogeniert sind oder eine bis drei Gruppen R^{c} tragen können:
R^{c} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino,
Di-C₁-C₆-alkylamino, C₂-C₆-Alkenyl, C₃-C₆-Alkenyloxy, C₃-C₆-Alkinyloxy oder C₁-C₄-Alkylendioxy, welches halogeniert sein kann; mit der Ausnahme von 3-[(4-Amino-2-hydroxy-benzoyl)-hydrazonomethyl)-2-hydroxy-benzoesäure.

2. Verbindungen der Formel I nach Anspruch 1, in der m Null bedeutet.

3. Verbindungen der Formel I nach Ansprüchen 1 oder 2, in der m 1 bedeutet und X in 4-Stellung steht.

4. Verbindungen der Formel I nach Ansprüchen 1 bis 3, wobei
A Methoxy, NH₂, NHCH₃ oder N(CH₃)₂; und
R² Wasserstoff oder Methyl
bedeuten.

5. Verbindungen der Formel I nach Ansprüchen 1 bis 4, in der R² Wasserstoff bedeutet.

6. Verfahren zur Herstellung von Verbindungen der Formel I nach Anspruch 1 durch Halogenierung von Salicylsäureestern der Formel II, 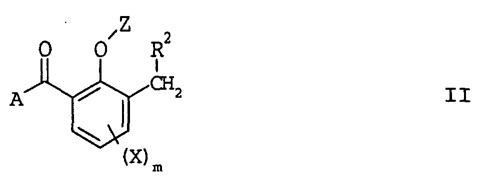 in der A für C₁-C₄-Alkoxy und Z für eine unter sauren Bedingungen abspaltbare Schutzgruppe steht, unter radikalischen Bedingungen zu Dihalogeniden der Formel III, 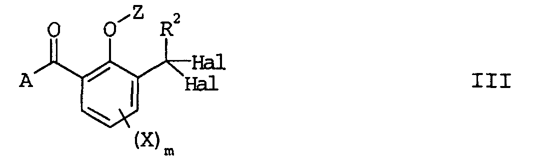 in der Hal für Chlor oder Brom steht, Oxidation und Abspaltung der Schutzgruppe von III unter sauren Bedingungen zu Verbindungen der Formel IV, 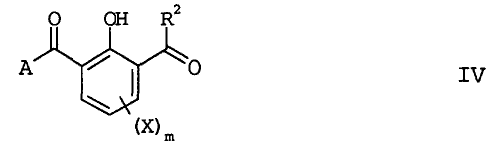 und, zur Herstellung von Verbindungen I, in denen A NH₂ oder N(CH₃)₂ bedeutet, Amidierung von IV mit Ammoniak oder Methylamin zu Salicylsäureamiden der Formel IV.1 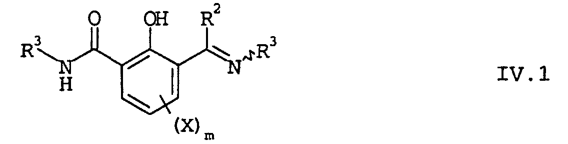 in der R³ für Wasserstoff oder Methyl steht, Abspaltung der Iminogruppe aus IV.1 zu Ketoverbindungen der Formel IV.2, 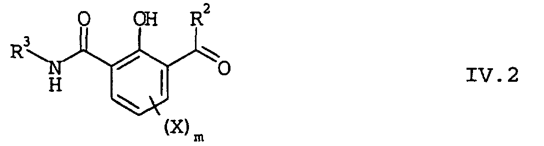 und, zur Herstellung von Verbindungen I, in denen A N(CH₃)₂ bedeutet, Amidierung von IV mit Dimethylamin zu Salicylsäureamiden der Formel IV.3, 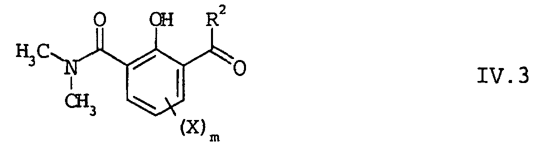 und, zur Herstellung von Verbindungen der Formel I, in der A für Hydroxy steht, Verseifung von IV zu Verbindungen IV.4, 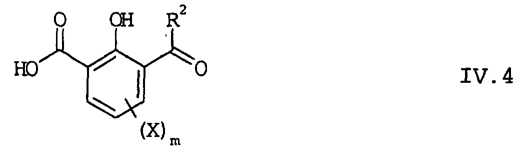 und Kondensation von IV, IV.2, IV.3 oder IV.4 mit Hydraziden der Formel V 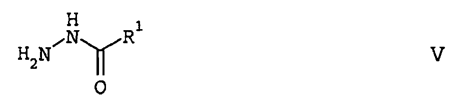 zu Verbindungen der Formel I.

7. Verfahren nach Anspruch 6, in dem in Formeln II und III Z für eine Acetylgruppe steht.

8. Verwendung der Verbindungen der Formeln IV, IV.2 und IV.3 als Zwischenprodukte in einem Verfahren nach Anspruch 6.

9. Zur Bekämpfung von pflanzenpathogenen Schadpilzen geeignetes Mittel, enthaltend einen festen oder flüssigen Trägerstoff und eine Verbindung der Formel I gemäß Anspruch 1.

10. Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

## Claims

1. A salicylic acid derivative of the formula I 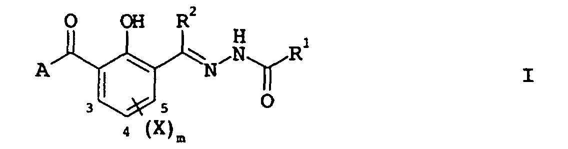 where the index and the substituents are as follows:
X is halogen, NO₂, cyano, C₁-C₄-alkyl or C₁-C₄-alkoxy;
m is 0, 1, 2 or 3, it being possible for the substituents X to differ from each other if m is greater than 1;
A is OH, C₁-C₄-alkoxy, NH₂, NHCH₃ or N(CH₃)₂;
R¹ is phenyl which is unsubstituted or substituted by one to three radicals R^{a}:
R^{a} is cyano, nitro, amino, aminocarbonyl, aminothiocarbonyl, halogen, hydroxyl, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, phenyl, phenoxy, benzyl, benzyloxy, 5- or 6-membered heterocyclyl, 5- or 6-membered hetaryl, 5- or 6-membered hetaryloxy, C(=NOR^{α})-OR^{β} or OC(R^{α})₂-C(R^{β})=NOR^{β}, the cyclic radicals, in turn, being unsubstituted or substituted by one to three radicals R^{b}:
R^{b} is cyano, nitro, halogen, hydroxyl, amino, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkenyl, phenyl, phenoxy, phenylthio, benzyl, benzyloxy, 5- or 6-membered heterocyclyl, 5- or 6-membered hetaryl, 5- or 6-membered hetaryloxy or C(=NOR^{α})-OR^{β}; R^{α},R^{β} is hydrogen or C₁-C₆-alkyl;
R² is hydrogen, cyano, C₁-C₆-alkyl, C₂-C₆-alkenyl, C₂-C₆-alkynyl, C₁-C₆-haloalkyl, C₁-C₆-alkoxy or C₁-C₆-alkylthio,
the hydrocarbon radicals being unsubstituted or partially or fully halogenated or it being possible for them to have attached to them one to three groups R^{c}:
R^{c} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyl, C₃-C₆-alkenyloxy, C₃-C₆-alkynyloxy or C₁-C₄-alkylenedioxy which can be halogenated; with the exception of 3-[(4-amino-hydroxybenzoyl)hydrazonomethyl]-2-hydroxybenzoic acid.

2. A compound of the formula I as claimed in claim 1 where m is zero.

3. A compound of the formula I as claimed in claim 1 or 2 where m is 1 and X is in the 4-position.

4. A compound of the formula I as claimed in any of claims 1 to 3 where
A is methoxy, NH₂, NHCH₃ or N(CH₃)₂; and
R² is hydrogen or methyl.

5. A compound of the formula I as claimed in any of claims 1 to 4 where R² is hydrogen.

6. A process for the preparation of compounds of the formula I as claimed in claim 1 by means of halogenation, under free-radical conditions, of salicylic esters of the formula II 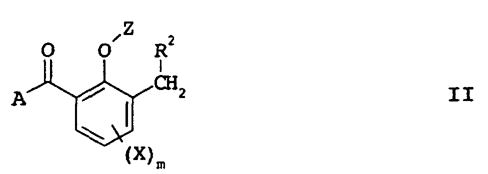 where A is C₁-C₄-alkoxy and Z is a protecting group which can be eliminated under acidic conditions, to give dihalides of the formula III 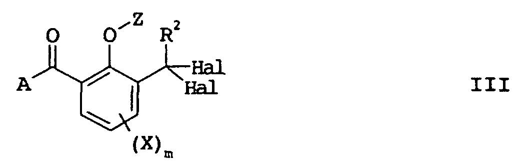 where Hal is chlorine or bromine, oxidation and elimination of the protecting group of III under acidic conditions to give compounds of the formula IV, 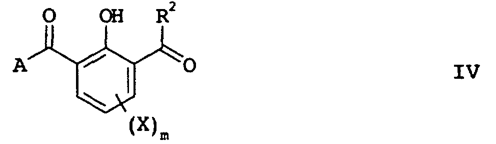 and, for the preparation of compounds I where A is NH₂ or N(CH₃)₂, amidation of IV with ammonia or methylamine to give salicylamides of the formula IV.1 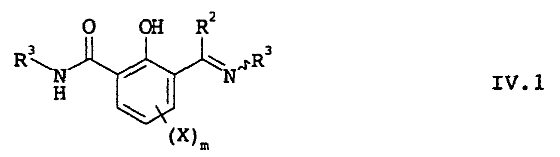 where R³ is hydrogen or methyl, elimination of the imino group from formula IV.1 to give keto compounds of the formula IV.2 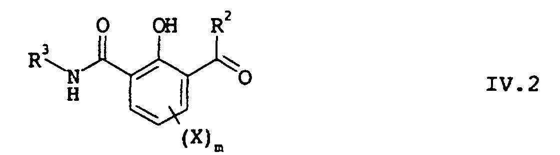 and, for the preparation of compounds I where A is N(CH₃)₂, amidation of IV with dimethylamine to give salicylamides of the formula IV.3, 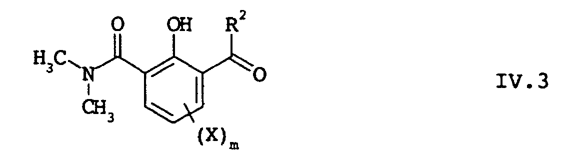 and, for the preparation of compounds of the formula I where A is hydroxyl, hydrolysis of IV to give compounds IV.4 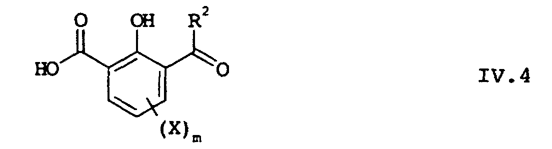 and condensation of IV, IV.2, IV.3 or IV.4 with hydrazides of the formula V 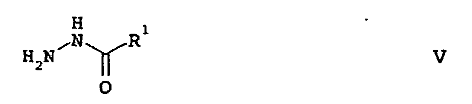 to give compounds of the formula I.

7. The process as claimed in claim 6 where, in formulae II and III, Z is an acetyl group.

8. The use of the compounds of the formulae IV, IV.2 and IV.3 as intermediates in a process as claimed in claim 6.

9. A composition which is suitable for controlling phytopathogenic harmful fungi, comprising a solid or liquid carrier and a compound of the formula I as claimed in claim 1.

10. A method of controlling phytopathogenic harmful fungi, which comprises treating the fungi or the materials, plants, the soil or seeds to be protected against fungal infection with an effective amount of a compound of the formula I as claimed in claim 1.

## Revendications

1. Dérivés d'acide salicylique de formule I, 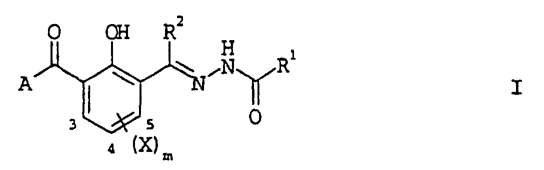 où l'indice et les substituants ont la signification suivante:
X halogéno, NO₂, cyano, alkyle en C₁-C₄ ou alcoxy en C₁-C₄;
m 0, 1, 2 ou 3, tandis que les substituants X peuvent être différents quand m est supérieur à 1;
A OH, alcoxy en C₁-C₄, NH₂, NHCH₃ ou N(CH₃)₂;
R¹ phényle, non-substitué ou substitué par un à trois restes R^{a}:
R^{a} cyano, nitro, amino, aminocarbonyle, aminothiocarbonyle, halogéno, hydroxy, alkyle en C₁-C₆, haogénoalkyle en C₁-C₆, alkyl(Cl-C6)carbonyle, alkyl(C₁-C₆)sulfonyle, alkyl(C₁-C₆)sulfoxyle, cycloalkyle en C₃-C₆, alcoxy en C₁-C₆, halogénoalcoxy en C₁-C₆, alkyl(C₁-C₆)oxycarbonyle, alkyl(C₁-C₆)thio, alkyl(C₁-C₆)amino, dialkyl(C₁-C₆)amino, alkyl(C₁-C₆)-aminocarbonyle, dialkyl(C₁-C₆)aminocarbonyle, alkyl(C₁-C₆)aminothiocarbonyle, dialkyl(C₁-C₆)aminothiocarbonyle, alcényle en C₂-C₆, alcényloxy en C₂-C₆, phényle, phénoxy, benzyle, benzyloxy, hétérocyclyle à 5 ou 6 chaînons, hétaryle à 5 ou 6 chaînons, hétaryloxy à 5 ou 6 chaînons, C(=NOR^{α})-OR^{β} ou OC(R^{α})2-C(R^{β})=NOR^{β}, tandis que les restes cycliques sont à leur tour non-substitués ou substitués par un à trois restes R^{b}:
R^{b} cyano, nitro, halogéno, hydroxy, amino, aminocarbonyle, aminothiocarbonyle, alkyle en C₁-C₆, halogénoalkyle en C₁-C₆, alkyl(C₁-C₆)sulfonyle, alkyl(C₁-C₆)sulfoxyle, cycloalkyle en C₃-C₆, alcoxy en C₁-C₆, halogénoalcoxy en C₁-C₆, alcoxy(C₁-C₆)carbonyle, alkyl(C₁-C₆)thio, alkyl-(C₁-C₆)amino, dialkyl(C₁-C₆)amino, alkyl-(Cl-C6)aminocarbonyle, dialkyl(C₁-C₆)aminocarbonyle, alkyl(C₁-C₆)aminothiocarbonyle, dialkyl(C₁-C₆)aminothiocarbonyle, alcényle en C₂-C₆, alcényloxy en C₂-C₆, cycloalkyle en C₃-C₆, cycloalcényle en C₃-C₆, phényle, phénoxy, phénylthio, 'benzyle, benzyloxy, hétérocyclyle à 5 ou 6 chaînons, hétaryle à 5 ou 6 chaînons, hétaryloxy à 5 ou 6 chaînons ou C(=NOR^{α})-OR^{β}; R^{α}, R^{β} hydrogène ou alkyle en C₁-C₆;
R² hydrogène, cyano, alkyle en C₁-C₆, alcényle en C₂-C₆, alcynyle en C₂-C₆, halogénoalkyle en C₁-C₆, alcoxy en C₁-C₆ ou alkyl(C₁-C₆)thio,
tandis que les restes hydrocarbonés sont non-substitués ou partiellement ou totalement halogénés ou peuvent porter un à trois groupe R^{c}:
R^{c} halogéno, cyano, nitro, hydroxy, alkyle en C₁-C₆, halogénoalkyle en C₁-C₆, alkyl(C₁-C₆)carbonyle, cycloalkyle en C₃-C₆, alcoxy en C₁-C₆, halogénoalcoxy en C₁-C₆, alcoxy(C₁-C₆)carbonyle, alkyl(C₁-C₆)thio, alkyl(C₁-C₆)amino, dialky(C₁-C₆)amino, alcényle en C₂-C₆, alcényloxy en C₃-C₆, alcynyloxy en C3-C6 ou alkylène(C₁-C₄)dioxy, qui peut être halogéné;
à l'exception de l'acide 3-[(4-amino-2-hydroxy-benzoyl)-hydrazonométhyl]-2-hydroxy-benzoïque.

2. Composés de formule I selon la revendication 1, où m vaut zéro.

3. Composés de formule I selon les revendications 1 ou 2, où m vaut 1 et X est en position 4.

4. Composés de formule I selon les revendications 1 à 3, où
A désigne le méthoxy, NH₂, NHCH₃ ou N(CH₃)₂; et
R² représente l'hydrogène ou le méthyle.

5. Composés de formule I selon les revendications 1 à 4, où R² désigne l'hydrogène.

6. Procédé pour la préparation de composés de formule I selon la revendication 1 par halogénation d'esters d'acide salicylique de formule II, 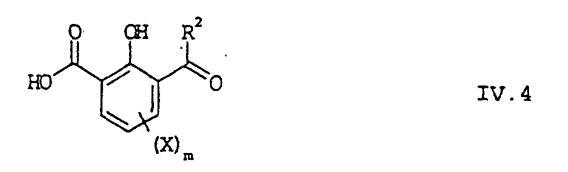 où A désigne un alcoxy en C₁-C₄ et Z représente un groupe protecteur éliminable dans des conditions acides, dans des conditions radicalaires pour donner des dihalogénures de formule III, 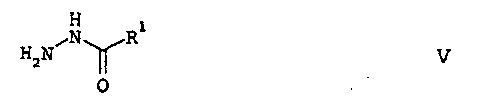 où Hal désigne le chlore ou le brome, oxydation et élimination du groupe protecteur de III dans des conditions acides pour donner des composés de formule IV, 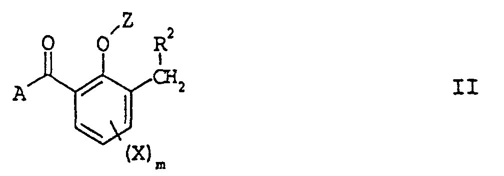 et, pour la préparation de composés I, dans lesquels A représente NH₂ ou N(CH₃)₂, amidation de IV avec de l'ammoniac ou de la méthylamine pour donner des amides d'acide salicylique de formule IV.1, 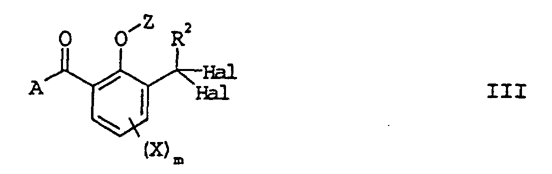 où R³ désigne l'hydrogène ou le méthyle, élimination du groupe imino de IV.1 pour donner des composés cétoniques de formule IV.2, 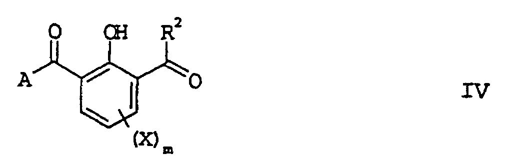 et, pour la préparation de composés I dans lesquels A représente N(CH₃)₂, amidation de IV avec de la diméthylamine pour donner des amides d'acide salicylique de formule IV.3, 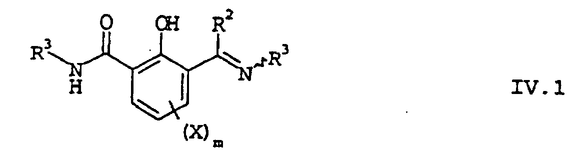 et, pour la préparation de composés de formule I dans laquelle A désigne l'hydroxy, saponification de IV en composés IV.4, 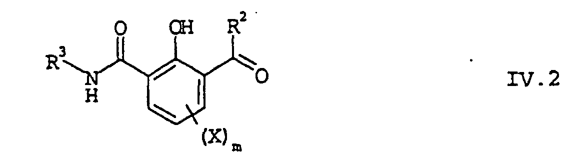 et condensation de IV, IV.2, IV.3 ou IV.4 avec des hydrazides de formule V 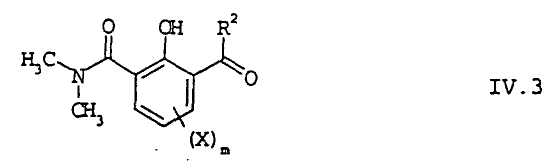 pour donner des composés de formule I.

7. Procédé selon la revendication 6, dans lesquels dans les formules II et III Z désigne un groupe acétyle.

8. Utilisation des composés de formules IV, IV.2 et IV.3 comme produits intermédiaires dans un procédé selon la revendication 6.

9. Produit approprié pour la lutte contre les champignons nuisibles phytopathogènes, contenant un support solide ou liquide et un composé de formule I selon la revendication 1.

10. Procédé pour la lutte contre les champignons nuisibles phytopathogènes, **caractérisé par le fait qu'**on traite les champignons ou les matériaux, plantes, sols ou semences à protéger contre une attaque par des champignons avec une quantité efficace d'un composé de formule générale I selon la revendication 1.
